# EUROPEAN PATENT APPLICATION

(11) **EP 2 018 883 A1**
(43) Date of publication of application: **28.01.2009**
(21) Application number: 07743102.1
(22) Date of filing: 10.05.2007
(51) Int. Cl.: A61M 5/158

(54) **INDWELLING NEEDLE ASSEMBLY**

(30) Priority: 17.05.2006 JP 2006138109
(71) Applicant: Terumo Kabushiki Kaisha, Tokyo 151-0072 (JP)
(72) Inventor: KOBAYASHI, Ryoji, Yamanashi, 4093853 (JP); OGAWA, Junichi c/o Terumo Kabushiki Kaisha, Yamanashi, 4093853 (JP); MURASHITA, Takato c/o Terumo Kabushiki Kaisha, Nakakoma-gun, Yamanashi, 4093853 (JP)
(74) Representative: TBK-Patent
(86) International application number: PCT/JP2007/059667
(87) International publication number: WO 2007/132732

(57) **Abstract**

An indwelling needle assembly has an inner needle having a sharp point at its tip, an inner needle hub fixed to the base end of the inner needle, a hollow outer needle in which the inner needle is inserted, and a seal section provided in the outer needle hub. In the indwelling needle assembly, the seal section has open/close means having a seal section body with a hole into which the inner needle is insertable, a shutter member for opening and closing the hole, and an urging member for urging the shutter member in the direction to close the hole.

## Description

### TECHNICAL FIELD

The present invention relates to an indwelling needle assembly, which is made to puncture a blood vessel and left dwelling within the blood vessel, in the case of an infusion, for example.

### BACKGROUND ART

In the case that an infusion is carried out on a patient, or in other similar cases, an indwelling needle, which is connected to an infusion line, is made to puncture a blood vessel of the patient and is left dwelling within the blood vessel.

Such an indwelling needle includes a hollow outer needle, an outer needle hub fixed to a base end of the outer needle, an inner needle having a sharp point at a tip portion thereof and which is inserted into the outer needle, and an inner needle hub fixed to the base end of the inner needle (refer to, for example, Patent Document 1).

In the case of puncturing a patient's blood vessel with the indwelling needle, a puncturing operation is conducted in an assembled condition in which the inner needle is inserted into the outer needle and the point of the inner needle protrudes from the tip of the outer needle. In the assembled condition, usually, a connector of an infusion line is placed in connection with the outer needle hub.

When the point of the inner needle arrives inside of the blood vessel, blood that has flowed in through an aperture at the needle point flows through a lumen of the inner needle and into the interior of a transparent inner needle hub (flashback). This makes it possible to confirm (visually check) that the inner needle has captured (securely punctured) the blood vessel.

Upon confirmation of flashback, the outer needle is advanced and is inserted into the blood vessel.

Next, while holding the outer needle by hand, the inner needle is pulled out of the outer needle. Then, an infusion liquid is fed through the infusion line and the outer needle, which are connected to each other.

Meanwhile, a seal member (plug body) is provided in (fixed to) the outer needle hub. The seal member can be pierced by the inner needle and has a self-closing property, so that the pierced hole thereof closes by itself upon evulsion of the inner needle after having punctured the seal member.

However, the indwelling needle having the aforementioned seal member has a problem in that, in cases where a condition persists in which the seal member is pierced by the inner needle (assembled condition) for an extended period of time, a semi-permanent condition, which is established by the inner needle, is imparted to the seal member, possibly resulting in the pierced hole not becoming closed upon evulsion of the inner needle from the seal member. In other words, the sealing performance (sealing function) of the seal member is lowered, so that a liquid, such as blood or a liquid medicine, may leak out through the pierced hole.
Patent Document 1:
Japanese Laid-Open Patent Publication No. 10-179734

### DISCLOSURE OF THE INVENTION

It is an object of the present invention to provide an indwelling needle assembly, in which a liquid can be securely prevented from flowing out from a base end of an outer needle hub, in a disassembled condition where an inner needle has been evulsed from an outer needle.

In order to attain the above object, according to the present invention, there is provided an indwelling needle assembly including:
an inner needle having a sharp needlepoint at a tip portion thereof;
an inner needle hub fixed to a base end part of the inner needle;
a hollow outer needle in which the inner needle is inserted;
an outer needle hub fixed to a base end part of the outer needle and having a joint section to which a tube is connected; and
   a seal section provided in the outer needle hub, wherein the seal section includes:
a seal section body having a hole into which the inner needle can be inserted; and
opening/closing means having a shutter member for opening and closing the hole, and an urging member for urging the shutter member in a direction to close the hole.

This ensures that a liquid can be securely prevented from flowing out from the base end of the outer needle hub in a disassembled condition, where the inner needle has been evulsed from the outer needle.

In addition, in the indwelling needle assembly according to the present invention, preferably, the shutter member is provided in a space formed at an intermediate part of the hole.

This ensures that the shutter member can be displaced between a first position, to produce an open condition in which the hole is open so that the inner needle can be inserted into the hole, and a second position in which the inner needle, when evulsed from the hole, is partially contained within the hole so as to close the hole. Therefore, the shutter member is displaced and assumes the second position when the disassembled condition is obtained, so that liquid can be more securely prevented from flowing out from the base end of the outer needle hub.

Further, in the indwelling needle assembly according to the present invention, preferably, the seal section body has a recess, which is provided to confront the hole and to face the shutter member, and the shutter member is partially contained within the recess when the hole is in a closed state.

This ensures that, in the disassembled condition, the hole in the seal section body is more securely closed with the shutter member, so that liquid, such as blood or a liquid medicine, can be more securely prevented from flowing out from the base end of the outer needle hub by way of the hole.

In addition, in order to attain the above object, according to the present invention, an indwelling needle assembly is provided, including:
an inner needle having a sharp needlepoint at a tip portion thereof;
an inner needle hub fixed to a base end part of the inner needle;
a hollow outer needle in which the inner needle is inserted;
an outer needle hub fixed to a base end part of the outer needle and having a joint section to which a tube is connected; and
a seal section provided in the outer needle hub, wherein the seal section includes:
   a seal section body having a hole into which the inner needle can be inserted; and
   opening/closing means provided in the seal section body and having a deformable section for opening and closing the hole through deformation, and an urging member for urging the deformable section in a direction to close the hole.

This ensures that liquid can be securely prevented from flowing out from the base end of the outer needle hub, in a disassembled condition in which the inner needle has been evulsed from the outer needle.

In addition, the indwelling needle assembly according to the present invention preferably further includes a pushing member for pushing the deformable section, between the deformable section and the urging member, wherein the deformable section is pushed by the urging member through the pushing member.

This ensures, in the case where the pushing member is formed by a hard material, that the deformable section can be deformed more securely.

In addition, in the indwelling needle assembly according to the present invention, preferably, the seal section body has a recess, which is provided to confront the hole and to face the deformable section, and the deformable section is partially contained within the recess when the hole is in a closed state.

This ensures that, in a disassembled condition, the hole in the seal section body is more securely closed by the deformable section, so that liquid such as blood or a liquid medicine can be more securely prevented from flowing out from the base end of the outer needle hub by way of the hole.

Further, in the indwelling needle assembly according to the present invention, preferably, the outer needle hub has a containing section therein for containing at least a part of the urging member.

This ensures that, in the case that the outer needle hub is formed by a hard material, a pushing force (urging force) supplied by the urging member can be more securely exerted on the shutter member.

In addition, in the indwelling needle assembly according to the present invention, preferably, the urging member includes an elastically deformable spring.

This ensures that a pushing force (urging force) can be more securely applied to the shutter member.

Further, in the indwelling needle assembly according to the present invention, preferably, the spring is a coil spring.

This ensures that a semi-permanent bend or tendency to bend is less liable to be produced, and thus the opening/closing means can be more securely operated, when required.

In addition, in the indwelling needle assembly according to the present invention, preferably, the seal section body is made of an elastic material.

This ensures that the seal section body has a self-closing property, so that in both the assembled condition and in the disassembled condition, liquid such as blood or a liquid medicine can be securely prevented from flowing out from the base end of the outer needle hub.

### BRIEF DESCRIPTION OF THE DRAWINGS

FIG. 1 is a perspective view showing a first embodiment of an indwelling needle assembly according to the present invention;
FIG. 2 is a sectional view taken along line A-A of FIG. 1;
FIG. 3 is a sectional view taken along line A-A of FIG. 1;
FIG. 4 is a sectional view taken along line A-A of FIG. 1;
FIG. 5 is a sectional view taken along line A-A of FIG. 1;
FIG. 6 is a perspective view, corresponding to FIG. 5, of the indwelling needle assembly shown in FIG. 1;
FIG. 7 is a perspective view of the indwelling needle assembly shown in FIG. 1, showing a condition in which a tube has been detached from an inner needle hub;
FIG. 8 is a longitudinal sectional view showing, in an enlarged form, a tip part of an outer needle hub, which is possessed by an indwelling needle assembly according to a second embodiment of the invention; and
FIG. 9 is a longitudinal sectional view showing, in an enlarged form, a tip part of an outer needle hub, which is possessed by the indwelling needle assembly according to the second embodiment.

### BEST MODE FOR CARRYING OUT THE INVENTION

An indwelling needle assembly according to the present invention will be described in detail below, based on preferred embodiments thereof as shown in the attached drawings.

### <First Embodiment>

Initially, a first embodiment of the indwelling needle assembly according to the present invention will be described.

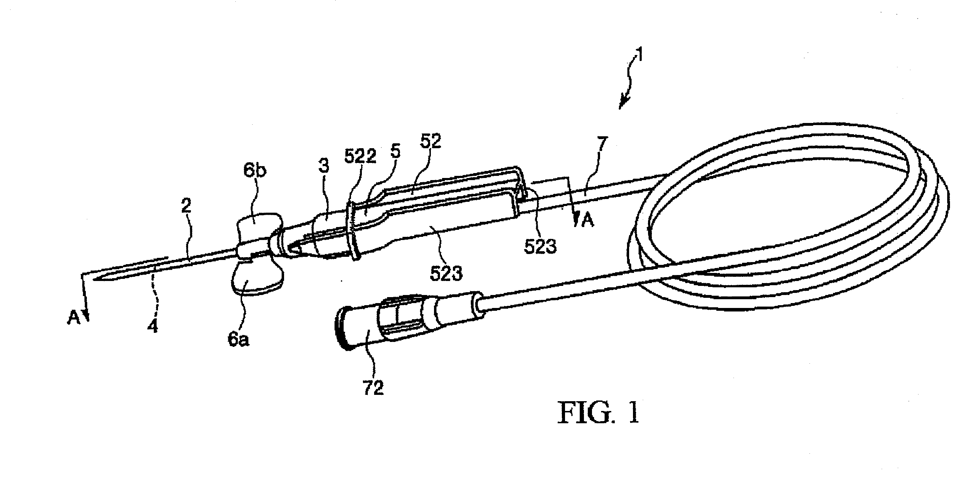

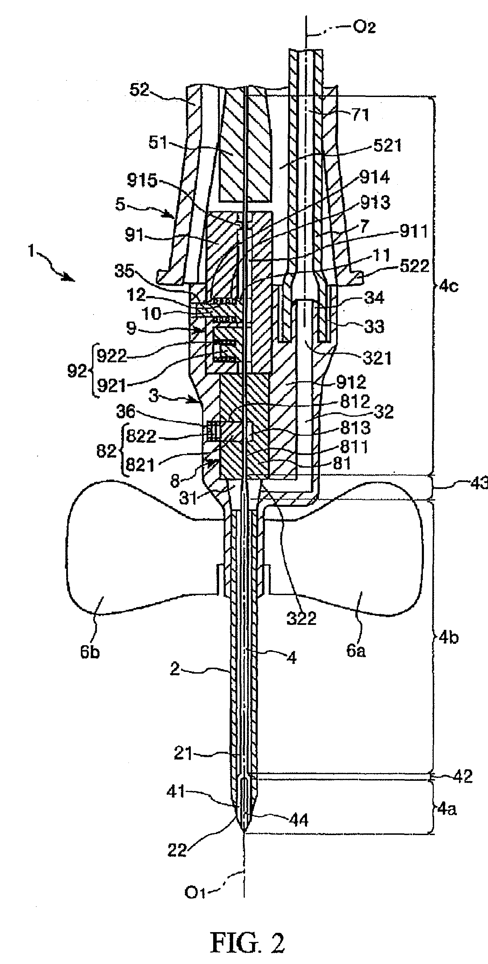

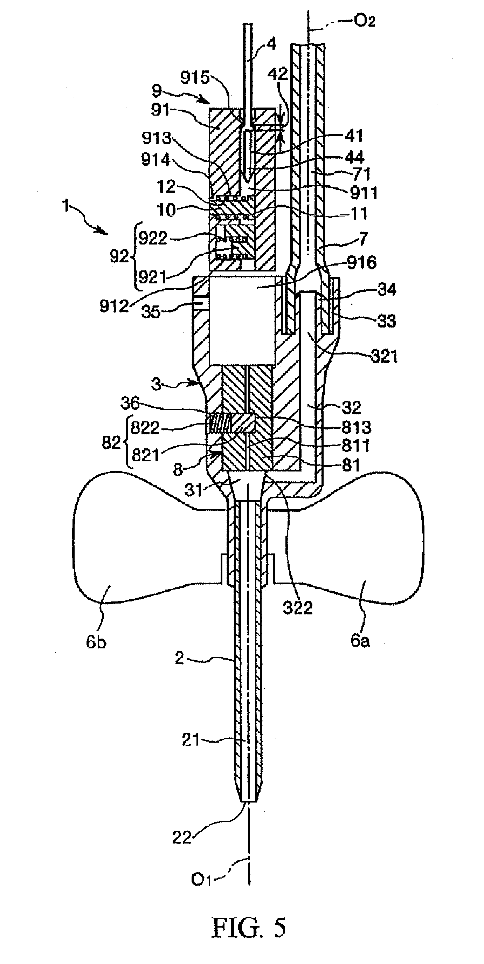

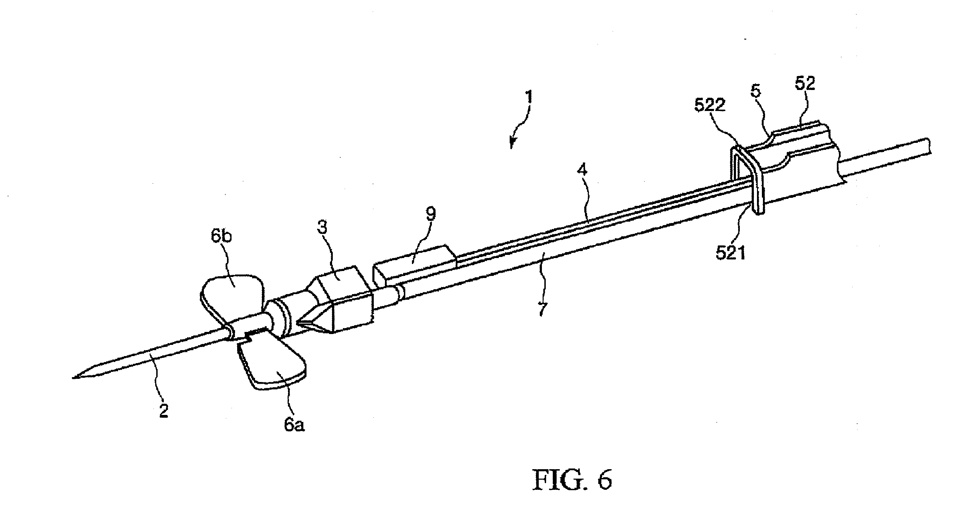

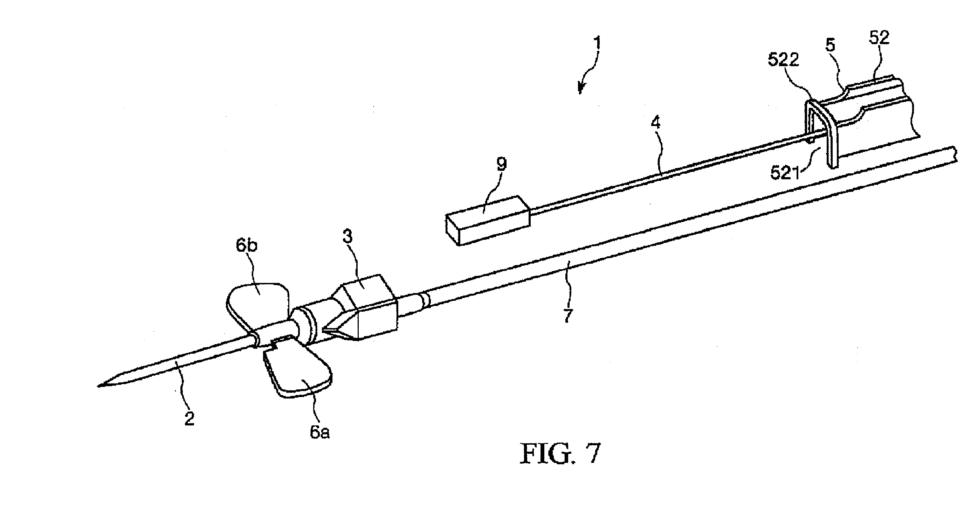

FIG. 1 is a perspective view of a first embodiment of the indwelling needle assembly according to the present invention, FIGS. 2 to 5 are sectional views taken along line A-A of FIG. 1, FIG. 6 is a perspective view, corresponding to FIG. 5, of the indwelling needle assembly shown in FIG. 1, and FIG. 7 is a perspective view of the indwelling needle assembly shown in FIG. 1, showing a condition in which a tube has been detached from an inner needle hub.

Incidentally, in the following description, the right side in FIGS. 1, 6 and 7 will be referred to as "the base end," and the left side as "the tip." Similarly, the upper side in FIGS. 2 to 5 will be referred to as "the base end," and the lower side as "the tip."

The indwelling needle assembly 1, as shown in the drawings, includes a hollow outer needle 2, an outer needle hub 3 fixed to a base end part of the outer needle 2, an inner needle 4 inserted in the outer needle 2, an inner needle hub 5 fixed to a base end part of the inner needle 4, and a tube 7 connected to a base end part (or a side part) of the outer needle hub 3, so that the lumen 71 thereof communicates with the lumen 21 of the outer needle 2.

The indwelling needle assembly 1 can assume the state shown in FIG. 2 (this state also is shown in FIG. 1) (hereinafter this state will be referred to as an "assembled condition"), and the state shown in FIG. 5 (this state also is shown in FIGS. 6 and 7) (hereinafter this state will be referred to as a "disassembled condition"). The assembled condition is a condition in which the inner needle 4 is inserted into the outer needle 2, and the inner needle hub 5 and the outer needle hub 3 abut against each other. Further, the disassembled condition is a condition in which the inner needle 4 has been evulsed from the outer needle 2, whereby the indwelling needle assembly 1 is disassembled into two structures (a structure on the side of the inner needle 4, and a structure on the side of the outer needle 2).

Configurations of components of the indwelling needle assembly 1 shall be described below.

The outer needle 2 preferably comprises a needle having a certain degree of flexibility. The material constituting the outer needle 2 preferably is a resin material, and more particularly, a flexible resin material. Specific examples of the material for the outer needle 2 include fluororesins such as PTFE, ETFE, PFA, etc., olefin resins such as polyethylene, polypropylene, etc., and mixtures thereof, polyurethane, polyesters, polyamides, polyether-nylon resins, and mixtures of olefin resin with ethylene-vinyl acetate copolymer.

The aforementioned outer needle 2 preferably is formed such that the entirety or a portion thereof permits the inside to be seen from the exterior. Specifically, the outer needle 2 preferably is formed from a transparent (colorless transparent), colored transparent, or semi-transparent resin. This ensures that when a blood vessel is captured by the outer needle 2, flashback of blood flowing in through a tip aperture 22 of the outer needle 2 can be confirmed visually.

In addition, the material constituting the outer needle 2 may be admixed with a radiopaque agent, such as barium sulfate, barium carbonate, bismuth carbonate, tungstic acid, etc., in order to obtain a contrast function.

The outer needle hub 3 is firmly attached (fixed) in a liquid-tight manner to a base end part of the outer needle 2 by way of caulking, welding (heat welding, high-frequency welding, etc.), adhesion with an adhesive, or the like.

The outer needle hub 3 is composed of a substantially tubular member. The inside 31 of the outer needle hub 3 communicates with the lumen 21 of the outer needle 2.

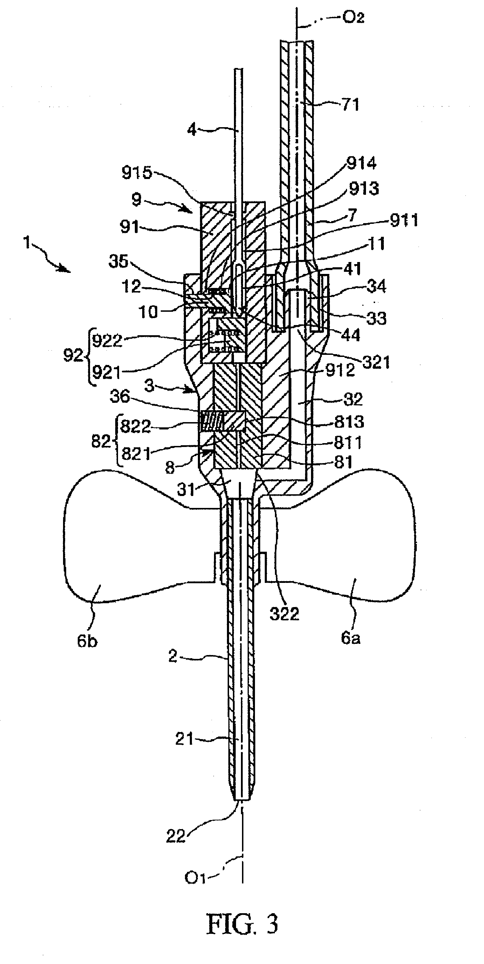

The outer needle hub 3 is provided, in a right side wall part thereof as shown in FIG. 2 (as well as in FIGS. 3 to 5), with a passage 32, one end of which opens into the inside 31 of the outer needle hub 3 via an aperture 322. The passage 32 is substantially L-shaped. The other end of the passage 32 opens at a recess 33, which is provided in a recessed form in the base end of the outer needle hub 3, and is provided with an aperture 321. Further, at a tip surface (bottom surface) of the recess 33, an annular projecting part (joint section) 34 is formed, so as to surround the aperture 321 and to protrude in the direction of the base end. In other words, the passage (lumen) in the projecting part (joint section) 34 communicates with the inside 31 of the outer needle hub 3.

The projecting part 34 is inserted into the lumen 71 of a tip part of the tube 7, whereby one end part (tip part) of the tube 7 is connected to the outer needle hub 3. This ensures that a liquid, such as a liquid medicine, can be supplied into the outer needle 2 (outer needle hub 3) by way of the tube 7.

Further, as shown in FIG. 2 (as well as in FIGS. 3 to 5), on the left and right sides of the outer needle hub 3, a pair of wings 6a and 6b are formed integrally with the outer needle hub 3 as an operating section. The wings 6a, 6b are flexible, and can be opened and closed by bending or curving portions thereof near the joint sections where the wings 6a, 6b are joined to the outer needle hub 3.

When a blood vessel or the like is punctured with the outer needle 2 and the inner needle 4, the wings 6a and 6b are pinched and placed in a closed state, and an operation for moving the inner needle 4 and the outer needle 2 along the longitudinal direction thereof, i.e., a puncturing operation, can be performed. When the outer needle 2 is left in an indwelling state, the wings 6a and 6b are placed in an opened state, and the wings 6a and 6b are fixed to the skin using a pressure sensitive adhesive tape or the like.

In the assembled condition shown in FIGS. 1 and 2, the inner needle 4, having a sharp needle point 41 at the tip end thereof, is inserted into the outer needle 2.

The length of the inner needle 4 is set such that, in an assembled condition, at least the point 41 thereof protrudes from the tip aperture 22 of the outer needle 2.

The inner needle 4 may be a hollow needle, but preferably, the inner needle 4 is a solid needle. With the inner needle 4 formed as a solid needle, a sufficient strength can be secured, while the outer diameter of the inner needle 4 can be set to a small value. In addition, when the inner needle 4 is a solid needle, high safety is ensured, because there is no danger of blood remaining inside the inner needle 4, or of blood flowing out from the inner needle 4 at the time that the inner needle 4 is discarded, after an operation thereby has been completed.

Further, when the inner needle 4 is a hollow needle, blood flows into a hollow part of the inner needle 4 upon puncturing a blood vessel with the inner needle 4, whereupon flashback of the blood is confirmed. On the other hand, when the inner needle 4 is a solid needle, blood flows into the gap formed between the inner needle 4 and the outer needle 2, so that flashback of blood can be confirmed earlier and more speedily.

Incidentally, although the inner needle 4 may be configured to have both a hollow part and a solid part (for example, a portion of the lumen of a hollow needle is filled so that the needle is hollow at the tip side and solid on the base end side thereof), forming the inner needle 4 entirely from a single member enables a reduction in the cost of the inner needle 4.

In addition, the inner needle 4 has a plurality of sections (in this embodiment, three sections), each of which are different in outer diameter. Specifically, the inner needle 4 has a maximum outer diameter section 4a having a maximum outer diameter on the tip side (tip part), a minimum outer diameter section 4c having a minimum outer diameter on the base end side, and an intermediate outer diameter section 4b having an intermediate outer diameter between the maximum outer diameter section 4a and the minimum outer diameter section 4c.

Further, the inner needle 4 has a structure in which a first outer diameter varying section 42, which continuously varies in outside diameter, is formed at a boundary section between the maximum outer diameter section 4a and the intermediate outer diameter section 4b, and a second outer diameter varying section 43, which continuously varies in outer diameter, is formed between the intermediate outer diameter section 4b and the minimum outer diameter section 4c.

In each of the outer diameter varying sections 42 and 43, the outer diameter of the inner needle 4 may vary stepwise. However, when the outer diameter is set to vary continuously (in a tapered manner), the outer diameter varying sections 42 and 43 can be prevented from becoming caught on a tip edge part of a hole 811 in a seal section body 81 (described later), or from becoming caught on a tip edge part of an inner needle passage 911 in a protector body 91 (described later) or the like, when the inner needle 4 is evulsed from the outer needle 2, so that the operation of evulsing the inner needle 4 from the outer needle 2 can be carried out more smoothly and securely.

Incidentally, the outer diameter varying sections 42 and 43 may be formed at the time that the inner needle 4 is manufactured. Alternatively, steps carried out necessarily at the time of forming a groove 44 (described later) therein may be utilized.

In addition, the maximum outer diameter section 4a has an outer diameter set substantially equal to the inner diameter of the outer needle 2, so that the maximum outer diameter section 4a is placed in intimate contact with the inside surface of the outer needle 2, in a condition where the inner needle 4 is inserted into the outer needle 2. The maximum outer diameter section 4a (tip part) is provided on an outer peripheral part thereof with a groove (passage) 44, which extends along the longitudinal direction of the inner needle 4 in a recessed form. The groove 44 provides communication between the tip aperture 22 of the outer needle 2 and the inside 31 of the outer needle hub 3, in a condition (assembled condition) where the inner needle 4 is inserted into the outer needle 2. The groove 44 functions as a passage for blood (humor) when a blood vessel is punctured by the needle, for example. This ensures that flashback of the blood can reliably be confirmed.

Examples of materials constituting the inner needle 4 may include metallic materials such as stainless steel, aluminum, aluminum alloys, titanium, titanium alloys, etc.

The inner needle hub 5 is firmly attached (fixed) to a base end part of the inner needle 4. The inner needle hub 5 includes a fixing section 51 for fixing the inner needle 4, and a cover section 52 provided on the outer peripheral side of the fixing section 51. Preferably, the fixing section 51 and the cover section 52 are formed integrally as one body.

Further, in the assembled condition, the tube 7 is disposed between the fixing section 51 and the cover section 52. In other words, in the assembled condition, the tube 7 is inserted into the inner needle hub 5. As a result, the tube 7 is covered with the inner needle hub 5, so that the tube 7 can be prevented from obstructing the operation of the indwelling needle assembly 1.

In addition, the cover section 52 is provided with a pair of guides 523, 523 for guiding the tube 7 (see FIG. 1). Each guide 523 constitutes a side wall (side section) of the cover section 52, whereby the tube 7 is guided such that the center axis 02 at the tip part of the tube 7 is substantially parallel to the longitudinal direction of the inner needle hub 5 (the center axis 01 of the outer needle 2).

Further, once the inner needle 4 has been evulsed from the outer needle 2, the tube 7 can be detached from the inner needle hub 5 through the space (gap 521) provided between the guides 523 (see FIGS. 6 and 7).

Examples of methods for fixing the inner needle 4 to the inner needle hub 5 (fixing section 51) include fitting, caulking, welding, adhesion with an adhesive, etc., and combinations of these methods. Further, in the case that the inner needle 4 is hollow, sealing must also be performed in order to prevent back-flowing blood from flying out from the base end of the inner needle 4 upon puncturing of a blood vessel, for example.

In addition, as shown in FIG. 1 (as well as in FIGS. 2, 6 and 7), the inner needle hub 5 may be provided with a flange 522 on the outer circumference of the tip portion thereof. When the flange 522 is provided, an operation for evulsing the inner needle 4 from the outer needle 2, for example, can be carried out more easily and reliably, by placing one's fingers on the flange 522.

Each of the inner needle hub 5 and the outer needle hub 3, as mentioned above, preferably is composed of a transparent (colorless transparent), colored transparent, or semi-transparent resin, so that the inside thereof may be seen from the exterior. This ensures that when a blood vessel is captured by the outer needle 2, flashback of blood flowing in through the above-mentioned groove 44 formed in the inner needle 4 can be confirmed visually. Further, when the inner needle 4 is solid, blood which is flashbacked under the pressure inside of the blood vessel, for example, will flow entirely back through the groove 44, so that better visual confirmation of the blood can be obtained.

The materials constituting the outer needle hub 3, the inner needle hub 5, and the wings 6a, 6b are not particularly limited. Examples of such constituent materials include various resin materials, for example, polyolefins such as polyethylene, polypropylene, ethylene-vinyl acetate copolymer, etc., polyurethane, polyamides, polyesters, polycarbonate, polybutadiene, polyvinyl chloride, etc.

The tube 7 is flexible. One end of the tube 7 is connected to the base end part of the outer needle hub 3, as mentioned above. A connector 72 is attached to the other end (base end part) of the tube 7. For example, a connector attached to an end part of an infusion line, for supplying an infusion liquid (liquid medicine) with which a patient is to be dosed, a mouth part (tip part) of a syringe containing a liquid medicine, or the like, may be connected to the connector 72.

Incidentally, the materials constituting the tube 7 are not particularly limited. Examples of such materials include polyolefins such as polyethylene, polypropylene, ethylene-vinyl acetate copolymer, etc., polyvinyl chloride, polybutadiene, polyamides, polyesters, etc., among which polybutadiene is preferred in particular. When polybutadiene is used to form the tube 7, appropriate flexibility and chemical resistance, as well as excellent chemical adsorption preventative properties, can be obtained.

As shown in FIGS. 2 to 5, the indwelling needle assembly 1 is provided with a seal section 8 at the inside 31 of the outer needle hub 3 (within the outer needle hub 3).

The seal section 8 includes a seal section body 81 having a hole 811 therein in which the inner needle 4 can be inserted, together with an opening/closing means 82 for opening and closing the hole 811.

The seal section body 81 includes a substantially cylindrical member, which is provided in a substantially central part of the seal section body 81, with the hole 811 penetrating therethrough along the longitudinal direction. In addition, the seal section body 81 is fixed in a liquid-tight manner to the outer needle hub 3.

Examples of methods for fixing (firmly attaching) the seal section body 81 to the outer needle hub 3 include welding (heat welding, high-frequency welding, etc.), adhesion with an adhesive, etc.

In the present embodiment, the seal section body 81 may be formed by a hard material or by an elastic material.

In the case that the seal section body 81 is formed by a hard material, the outer diameter (diameter) of the hole 811 is set approximately equal to or slightly larger than the outer diameter of the maximum outer diameter section 4a of the inner needle 4.

On the other hand, in the case that the seal section body 81 is formed by an elastic material, a configuration preferably is provided whereby the hole 811 is closed upon evulsion of the inner needle 4 from the hole 811 (i.e., the hole 811 has a self-closing property). This ensures that in a disassembled condition, the passage in which the inner needle 4 has been inserted can be closed not only at the location of the opening/closing means 82 (described later), but also within the seal section body 81. Therefore, liquid such as blood or a liquid medicine can be more securely prevented from flowing out from the base end of the outer needle hub 3. In addition, in the assembled condition as well, leakage of liquid from the base end of the outer needle hub 3 can be prevented.

Moreover, in this case, preferably, the minimum outer diameter section 4c of the inner needle 4 is located within the hole 811 in the assembled condition, as shown in FIG. 2. This ensures that a semi-permanent opening caused by the inner needle 4 can be prevented or restrained from being produced within the seal section body 81. Therefore, the sealing function (sealing performance) of the seal section body 81 can be prevented from being lowered.

The aforementioned hole 811 may be formed simultaneously with molding of the seal section body 81, or may be formed (machined) after molding of the seal section body 81 has been performed.

In the case that the seal section body 81 is formed by a hard material, materials similar to those constituting the outer needle hub 3, as described above, may be used as the hard material.

Further, in the case that the seal section body 81 is formed by an elastic material (i.e., a material having a self-closing property), examples of such an elastic material include various rubber materials such as natural rubber, isoprene rubber, butyl rubber, butadiene rubber, styrenebutadiene rubber, urethane rubber, nitrile rubber, acrylic rubber, fluoro-rubber, silicone rubber, etc., (particularly, vulcanized rubbers), various plastic elastomers based on urethane, polyester, polyamide, olefin, styrene or the like, and mixtures thereof, among which isoprene rubber is particularly preferred. When isoprene rubber is used to form the seal section body 81, a merit is provided in that compressive permanent strains are slight, and the service life of the product is prolonged.

The opening/closing means 82 includes a function for opening and closing (shutting off) an intermediate part of the hole 811, which has been pierced by the inner needle 4, in the disassembled condition.

The opening/closing means 82 according to the present embodiment includes a block-formed shutter member 821, formed as a body separate from the seal section body 81 and which operates to open and close the hole 811, and a coil spring (urging member) 822 for urging the shutter member 821 in a direction to close the hole 811.

The seal section body 81 is provided, on the left side wall portion thereof as shown in FIG. 2, with a through-hole 812, which penetrates through the seal section body 81 at an intermediate portion in the longitudinal direction. The shutter member 821 is contained (provided) within the through-hole (space) 812.

The shutter member 821 can be displaced between a first position (the position shown in FIG. 2), in which the shutter member 821 is in an open condition where the hole 811 is open so that the inner needle 4 can be inserted into the hole 811, and a second position (the position shown in FIGS. 3 to 5) corresponding to a closed condition where the inner needle 4, when evulsed from the hole 811, is partially contained within the hole 811 in order to close the hole 811.

As the material constituting the shutter member 821, the aforementioned elastic materials can suitably be used.

In addition, the inside surface of the outer needle hub 3 is provided with a recess (containing section) 36 therein that communicates with the through-hole 812 (in a part corresponding to the shutter member 821). The coil spring 822 is wholly or partially contained within the recess 36. The coil spring 822 abuts against the shutter member 821 (shutting-off member) on one end side thereof, and abuts against a bottom part of the recess 36 on the other end side thereof. The outer needle hub 3 normally is formed by a hard material. With such a configuration, therefore, a pushing force (urging force) generated by the coil spring 822 can securely be applied to the shutter member 821.

Examples of a spring which can be used as the coil spring 822 include springs produced by a method in which a metallic thin wire, such as superelastic alloy wires, piano wire, stainless steel wires, etc., or a thin wire made from a comparatively hard resin material or which has a restoring property, such as polyacetal, polyphenylene sulfide, polycarbonate, etc., is formed into a helical shape.

Incidentally, the coil spring 822 may or may not be fixed to the shutter member 821 at one end part thereof and/or to a bottom part of the recess 36 at another end part thereof.

In the assembled condition of the indwelling needle assembly 1 (i.e., in the condition shown in FIG. 2), the shutter member 821 primarily is withdrawn into the through-hole 812, so as to be located in the first position where the inner needle 4 can be inserted into the hole 811 in the seal section body 81, thereby resulting in an open condition in which the hole 811 is open. Further, in such an open condition, the coil spring 822 is pressed into a compressed state by the inner needle 4, and the shutter member 821 makes contact with the outside surface of the inner needle 4, while being restrained from moving toward the side of the hole 811.

On the other hand, when the inner needle 4 is evulsed from the hole 811 in order to obtain a disassembled condition (the condition shown in FIG. 5), restraint on movement of the shutter member 821 is released, and the coil spring 822 extends. This results in the shutter member 821 being moved into the second position toward the side of the hole 811, and the right surface of the shutter member 821 abuts (comes into firm contact) against the inside surface of the seal section body 81, thereby closing the hole 811 in a closed condition. Consequently, in the disassembled condition, liquid such as blood or a liquid medicine can securely be prevented from flowing out from the base end of the outer needle hub 3 by way of the hole 811 in the seal section body 8.

In addition, the hole 811 through which the inner needle 4 has passed is forcibly closed simultaneously with evulsion of the inner needle 4, so that a sealing mechanism can be realized, which is more stable and does not depend on the physical properties of the seal section body 81.

Further, the seal section body 81 is provided with a recess 813, which confronts the hole 811 and faces toward the shutter member 821. In the closed condition in which the hole 811 is closed, the shutter member 821 is partially contained within the recess 813. This ensures that, in a disassembled condition, the hole 811 in the seal section body 81 is more securely closed by the shutter member 821, so that liquid such as blood or a liquid medicine can more assuredly be prevented from flowing out, by way of the hole 811, from the base end of the outer needle hub 3. In addition, sterility at the interior (inside 31) of the outer needle hub 3 can be maintained more securely. Further, even when the inner needle 4 is unwillingly moved in the direction of the tip (in the distal direction), the shutter member 821 can be prevented from being pushed back into the first position by the inner needle 4.

Incidentally, although in the present embodiment, the opening/closing means 82 is disposed at a central part of the seal section 8, the opening/closing means 82 may also be provided at a tip part, or on a base end part, of the seal section 8.

In addition, the number of the opening/closing means 82 is not limited to being one. For example, a plurality of opening/closing means 82 may be provided. Furthermore, the number of the seal section(s) 8 is not limited to being one. For example, a plurality of seal sections 8 may be provided.

Further, in place of the coil spring 822, a V-shaped or a U-shaped leaf spring can also be used as the urging member. When an elastically deformable spring, formed by a filamentary body (or a belt-like body), is used, an urging force can be produced effectively within a narrow space, which contributes to a reduction in size. However, it should be noted that when the urging member is composed of the coil spring 822, a merit is provided in that the urging member is less liable to acquire a semi-permanent bend or a tendency to bend therein, so that the opening/closing means 82 can be more reliably operated when required.

In addition, preferably, at least one of the inside surface of the hole 811 and the outside surface (outer peripheral surface) of the inner needle 4 is subjected to a friction-reducing treatment, so as to reduce the frictional resistance between such surfaces.

The friction-reducing treatment is not particularly limited. Examples of such a treatment include a treatment in which a lubricant is applied to at least one of the inside surface of the hole 811 and the outside surface of the inner needle 4.

The lubricant as well is not particularly limited. Examples of suitable lubricants include silicone oil, polyethylene glycol, and polypropylene glycol.

When such a friction-reducing treatment is carried out, evulsion of the inner needle 4 from the seal section 8 can be performed more smoothly.

Moreover, the method for applying the lubricant is not particularly limited. Examples of such a method include a method in which a lubricant is applied to the inside surface of the hole 811 and/or the outside surface of the inner needle 4, and a method in which the seal section 8 and/or the inner needle 4 is immersed in a lubricant.

In addition, the indwelling needle assembly 1 has a protector 9 for covering at least the point 41 of the inner needle 4, upon evulsion of the inner needle 4 from the outer needle 2. The protector 9 will be described below.

As shown in FIG. 2 (as well as in FIGS. 3 to 5), the protector 9 includes a protector body 91 having a roughly parallelepiped outer shape, and a shutter means 92 provided inside the protector body 91.

The protector body 91 includes an inner needle passage 911 in a substantially central region thereof through which the inner needle 4 is passed in a penetrating form along the longitudinal direction of the protector body 91.

The inner needle passage 911 is roughly circular in cross-sectional shape, and the inner diameter thereof is set to be equal to or slightly larger than the outer diameter of the maximum outer diameter section 4a of the inner needle 4.

Further, an inside wall on the tip side of the protector body 91 (i.e., the surface that confronts the inner needle passage 911) is provided with a recess 912 therein.

The shutter means 92 is contained within the recess 912. The shutter means 92 includes a block-formed shutter member 921, and a coil spring (urging means) 922 for urging the shutter member 921 toward the side of the inner needle passage 911.

The shutter means 92 can be displaced between a first attitude (the attitude shown in FIG. 2), in which the shutter means 92 mostly is withdrawn into the recess 912 so that the inner needle 4 can be inserted into the inner needle passage 911, and a second attitude (the attitude shown in FIG. 3), in which a part of the shutter member 921 is contained within the inner needle passage 911 so as to inhibit passage of the point 41 of the inner needle 4.

When such a protector 9 is provided, the tip 41 of the used inner needle 4 can be covered rapidly and safely by a simple operation. In addition, by action of the shutter means 92, once the needle point 41 has been covered, the needle point 41 is prevented from protruding from the tip of the protector 9 (protector body 91). Therefore, when the inner needle 4 is discarded or in similar situations, it is possible to prevent an accident in which a worker punctures his finger or the like with the needle point 41 by mistake. Therefore, high safety is secured.

In addition, in the assembled condition, the protector 9 is substantially entirely covered by both the outer needle hub 3 and the inner needle hub 5. This ensures that the protector 9 does not obstruct the puncturing operation with the outer needle 2 and the inner needle 4, and therefore, the puncturing operation can be carried out more reliably. Incidentally, the protector 9 may be substantially entirely covered with either one of the outer needle hub 3 and the inner needle hub 5.

Furthermore, in the assembled condition, the protector 9 is located on the base end side relative to the seal section 8. This ensures that the protector 9 does not have to pass through the hole 811 in the seal section body 81 when the inner needle 4 is evulsed from the outer needle 2. Therefore, the operation can be carried out more easily and securely. Further, such a configuration permits the inner needle 4 to be shorter in overall length, which leads to a reduction in the size of the indwelling needle assembly 1, exclusive of the tube 7.

In addition, in the assembled condition, the aforementioned indwelling needle assembly 1 includes a fixing means for fixing the protector 9 to the outer needle hub 3, together with an engaging means (movement restricting means) for restricting movement of the inner needle 4 relative to the protector 9 in a direction opposite to the needle point 41, as a result of engagement between the inner needle 4 and the protector 9, in a condition where the protector 9 covers at least the point 41 of the inner needle 4. The fixing means and the engaging means will now be described in detail below.

### <Fixing Means>

First, the fixing means will be described.

The inside wall of the protector body 91 is provided with a through-hole 913 on the base end side of the recess 912, and a projecting part 914 that projects toward the inside is formed at the left end of the through-hole 913 as shown in FIG. 2.

A fixing pin 10 having a flange section 11 at the right end thereof as shown in FIG. 2 is inserted into the through-hole 913, in a condition where the coil spring 12 is contained therein. In this condition, the coil spring 12 abuts against the projecting part 914 at the left end thereof as shown in FIG. 2, and abuts against the flange section 11 at the right end thereof.

In addition, the outer needle hub 3 includes a through-hole 35 into which the fixing pin 10 can be inserted, provided on the base end of a left side wall portion thereof as shown in FIG. 2.

In a condition where the inner needle 4 is inserted (i.e., penetrates through) the inner needle passage 911, a right surface of the fixing pin 10 abuts against the outer peripheral surface (outside surface 45) of the inner needle 4, and a left end part of the fixing pin 10 protrudes from the through-hole 913, so as to be inserted into the through-hole 35. As a result, the protector 9 is fixed to the outer needle hub 3 (see FIGS. 2 and 3).

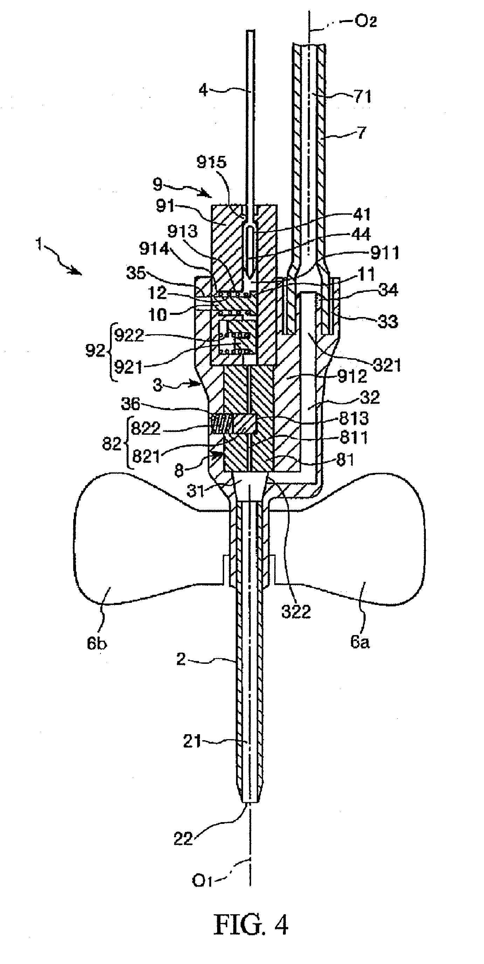

On the other hand, when the inner needle 4 is evulsed from the inner needle passage 911, the fixing pin 10 is pushed by the coil spring 12 and moves toward the right side in FIG. 4, and the left end part of the fixing pin 10 comes out of the through-hole 35. As a result, fixation of the protector 9 to the outer needle hub 3 is released (see FIG. 4).

Thus, in the present embodiment, the fixing means for fixing the protector 9 to the outer needle hub 3 is composed mainly of the through-hole 913, the fixing pin 10, the coil spring 12, and the inner needle 4.

In addition, as shown in FIG. 3, according to the present embodiment, the fixing means operates only after the shutter means 92 has been operated. Specifically, in a condition where the shutter means 92 is operated, fixation of the protector 9 to the outer needle hub 3 by the fixing means is maintained. Such a configuration ensures, in the condition where fixation of the protector 9 to the outer needle hub 3 is released, that the shutter means 92 can operate assuredly. Thus, it is possible to more securely prevent an accident in which a finger of a worker or the like is punctured by the needle point 41, when the inner needle 4 is discarded, or in other similar situations.

### <Engaging Means>

Next, the engaging means will be described.

At the base end part of the protector body 91, a reduced diameter section 915 is formed, in which the diameter of the inner needle passage 911 is reduced. The inner diameter of the reduced diameter section 915 is set greater than the outer diameter of the intermediate outer diameter section 4b and the minimum outer diameter section 4c of the inner needle 4, and smaller than the outer diameter of the maximum outer diameter section 4a.

This ensures that when the inner needle 4 is evulsed from the outer needle 2, the minimum outer diameter section 4c and the second outer diameter varying section 43, as well as the intermediate outer diameter section 4b, can pass through the reduced diameter section 915, whereas the first outer diameter varying section 42 cannot pass through the reduced diameter section 915 and becomes engaged with the reduced diameter section 915 (see FIG. 4).

In other words, according to the present embodiment, the first outer diameter varying section 42 and the reduced diameter section 915 constitute an engagement means for realizing engagement between the inner needle 4 and the protector 9.

When such an engaging means is provided, in a series of operations for evulsing the inner needle 4 from the outer needle 2, it is possible for the inner needle 4 to engage with the protector 9 and to release the protector 9 from the outer needle hub 3 (see FIGS. 4 and 5), and therefore, such operations can be carried out with extreme ease. In addition, the inner needle 4 can be prevented from coming out of the protector 9, which is in a state of covering the needle tip 41.

Further, the first outer diameter varying section 42 and the reduced diameter section 915 are formed respectively on the inner needle 4 and on the protector 9. This ensures a simple configuration, which does not increase the number of component parts, and contributes to reductions in both size and diameter.

Incidentally, other examples of the movement restricting means (drop-off preventive means), other than the above-mentioned engaging means, include a string having a predetermined length connecting the inner needle hub 5 and the protector 9, a foldable or contractible tubular body or a band-like body having a predetermined length connecting the inner needle hub 5 and the protector 9 so as to cover the inner needle 4, and a means based on engagement between a projecting part provided on one of the outside surface of the inner needle 4 and the inside wall of the inner needle passage 911, and a recess provided on the other member.

In the above-described indwelling needle assembly 1, as shown in FIGS. 1 to 5, the tube 7 is connected to the base end part of the outer needle hub 3, and, in the assembled condition, the center axis 01 of the outer needle 2 and the center axis 02 of the tube 7, at the tip portion thereof, are substantially in parallel with each other. In other words, the tube 7 protrudes toward the direction of the base end, from the base end of the outer needle hub 3.

The above-described configuration (i.e., a configuration in which the tube 7 protrudes in the direction of the base end of the outer needle hub 3 and is covered with the inner needle hub 5) produces the following effects.

First, since the tube 7 does not project toward a lateral side of the outer needle hub 3, when puncturing is carried out by the outer needle 2 and the inner needle 4, the outer needle hub 3 can be prevented from being pulled sideways by the tube 7 and losing balance, thereby making it difficult to perform the evulsing operation.

Secondly, since the tube 7 does not project toward the upper side of the outer needle hub 3, the tube 7 can be prevented from being sharply bent (i.e., from kinking) when the outer needle hub 3 is affixed to a patient, in the case that the outer needle 2 is left to dwell within a patient's blood vessel or the like.

Thirdly, since the tube 7 does not project toward a lateral side or toward the upper side of the outer needle hub 3, it is unnecessary to avoid the tube 7 so as not to pinch the tube 7, and it is easy to hold the inner needle hub 5 while only the outer needle 2 is advanced into a blood vessel after the outer needle 2 has entered into the blood vessel.

From the above-noted points of view, the indwelling needle assembly 1 also is excellent in operability.

Next, one example of a method for using the indwelling needle assembly 1 (in the case of puncturing a blood vessel) will be described in detail below.
[1] The indwelling needle assembly 1 is placed in the assembled condition, and a connector, which is attached to an end part of an infusion line, is preliminarily connected to the connector 72, thus enabling an infusion liquid to be supplied from the infusion line.
   Incidentally, in this instance, a predetermined portion of the tube 7 or the infusion line is pinched, for example, by a clamp (which forms one example of a passage opening/closing means), so as to preliminarily close the lumen of the tube 7 or the infusion line.
[2] Next, the closed state of the tube 7 or the infusion line by the clamp is released, whereupon the infusion liquid supplied from the infusion line is introduced through the tube 7 into the outer needle hub 3.
   The infusion liquid introduced into the outer needle hub 3 fills the passage 32, and is introduced into the lumen 21 of the outer needle 2, whereby the lumen 21 of the outer needle 2 is primed with the infusion liquid. In this case, a portion of the infusion liquid flows out via the tip aperture 22 of the outer needle 2. Further, in this case, the inner needle 4 and the seal section body 81 are in intimate contact with each other owing to the aforementioned configuration, and therefore, the infusion liquid can be prevented from flowing out from the base end of the outer needle hub 3.
[3] When priming is completed in this manner, the tube 7 or the infusion line is again closed with the clamp or the like. Then, the wings 6a and 6b are closed by pinching them between the fingers, whereupon the outer needle 2 and the inner needle 4 in an integrated state are made to puncture a patient's blood vessel (vein or artery), while using the wings 6a and 6b as a holding section (operating section).
   While puncturing of the blood vessel is thus carried out by holding the wings 6a and 6b, the puncturing angle is made smaller, i.e., the outer needle 2 and the inner needle 4 are held more closely in parallel in relation to the blood vessel, as compared to a case in which the puncturing operation is conducted by holding the outer needle hub 3 directly. Accordingly, the puncturing operation is easy to carry out, and the burden imposed on the patient's blood vessel is lessened.
   When a blood vessel is captured by the outer needle 2, the internal pressure inside the blood vessel (blood pressure) causes the blood to flow back in the direction toward the base end (in the proximal direction) through the groove 44 of the inner needle 4, and through the lumen 21 of the outer needle 2. Therefore, backflow of blood can be confirmed in at least one of the outer needle 2, the outer needle hub 3, the inner needle hub 5, and the tube 7, which have a property for permitting external visual confirmation.
   After such confirmation, the outer needle 2 and the inner needle 4 are further advanced by a minute distance in the direction of the tip.
   Further, at the time of puncturing the blood vessel (during centesis), the lumen 21 of the outer needle 2 has been primed with the infusion liquid, so that accidental penetration of bubbles into the blood vessel can reliably be prevented from occurring, and extremely high safety is secured.
   In addition, as mentioned above, a configuration is adopted in which the tube 7 is connected to the base end part of the outer needle hub 3. Further, in the assembled condition, the center axis O1 of the outer needle 2 and the center axis 02 of the tube 7 at the tip portion thereof are substantially parallel to each other. This ensures that the tube 7 does not form an obstacle at the time puncturing is carried out with the outer needle 2 and the inner needle 4, and thus, operability of the indwelling needle assembly 1 is enhanced.
[4] After the blood vessel has been captured by the outer needle 2, the outer needle 2 or the outer needle hub 3 is grasped and fixed by one hand, while the inner needle hub 5 is held by the other hand and pulled in the direction of the base end in order to evulse the inner needle 4 from the outer needle 2.
[5] When the inner needle 4 is further moved in the direction of the base end and the needle point 41 has passed through the hole 811 in the seal section body 81, the coil spring 822, deformation of which has been restricted by the inner needle 4, extends. In addition, under a pushing action by the coil spring 822, the shutter member 821 is moved from the first position (see FIG. 2) toward the side of the hole 811, so as to reach the second position (see FIGS. 3 and 5). When the shutter member 821 is placed in the second position, the right surface of the shutter member 821 comes into firm contact against the inside surface of the seal section body 81, whereby the hole 811 is forcibly closed, thereby performing a sealing function. This ensures that leakage of liquid through the hole 811 does not occur, and sterility in the outer needle hub 3 and the infusion line is secured.
[6] When the inner needle 4 is further moved in the direction of the base end and the needle point 41 passes through the vicinity of the recess 912 of the inner needle passage 911, the shutter member 921 is pushed by the coil spring 922 and is moved toward the side of the inner needle passage 911, whereupon the right surface of the shutter member 921 comes into abutment against the surface of the inner needle passage 911 opposed to the recess 912. In other words, the shutter means 92 is shifted from the first attitude (see FIG. 2) to the second attitude (see FIG. 3).
   When the shutter means 92 is placed in the second attitude, the shutter member 921 shuts off the inner needle passage 911. Therefore, even if the needle point 41 tends to return toward the direction of the tip, the needle point 41 cannot be returned, because the needle point 41 abuts against the shutter member 921.
[7] When the inner needle 4 is further moved in the direction of the base end and the needle point 41 passes through the vicinity of the through-hole 913 of the inner needle passage 911, the fixing pin 10 is pushed by the coil spring 12 and is moved toward the side of the inner needle passage 911, whereupon the right surface of the fixing pin 10 comes into abutment against the surface of the inner needle passage 911 opposed to the through-hole 913. In this instance, a left end part of the fixing pin 10 comes out of the through-hole 35 in the outer needle hub 3. As a result, fixation of the protector 9 to the outer needle hub 3 is released (see FIG. 4).
   In a condition where the fixed state of the protector 9 to the outer needle hub 3 is released, the shutter means 92 still operates assuredly. Therefore, it is possible to more securely prevent an accident, in which a worker punctures his finger or the like with the needle tip 41 by mistake when the inner needle 4 is discarded, or in other similar situations.
[8] When the inner needle 4 is further moved in the direction of the base end, the first outer diameter varying section 32 engages with the reduced diameter section 915 (i.e., the inner needle 4 engages with the protector 9) due to the fact that the first outer diameter varying section 32 cannot pass through the reduced diameter section 915.
   When the inner needle hub 5 is further pulled in the direction of the base end under this condition, the protector 9 in engagement with the inner needle 4 is moved in the direction of the base end together with the inner needle 4, so as to separate from the outer needle hub 3 (see FIG. 5).
   Incidentally, at the time of performing the series of operations in the aforementioned steps [5] through [7], according to the present embodiment, such operations can be carried out smoothly and assuredly, since the center axis 01 of the outer needle 2 and the center axis 02 of the tube 7 on the tip side thereof are kept substantially in parallel with each other, under the function of the guides 523 of the inner needle hub 5.
[9] Next, the tube 7 inserted in the inner needle hub 5 is detached through the gap 521 (see FIG. 7).
   After the inner needle 4 has been evulsed from the outer needle 2, the inner needle 4 and the inner needle hub 5 are no longer useful and hence are discarded.
   The inner needle 4 has the point 41 thereof covered with the protector 9, and in particular, the point 41 will not move toward the tip side beyond the shutter means 92 so as to protrude from the tip of the protector 9. This prevents an accident, in which a worker who is in charge of carrying out a discarding treatment or the like might puncture his finger with the needle point 41 by mistake.
[10] Subsequently, the wings 6a and 6b are opened and affixed to the skin with a pressure sensitive adhesive tape or the like, whereupon closure of the tube 7 or an infusion line by the clamp is released, in order to start supply of the infusion liquid.

The infusion liquid supplied from the infusion line is fed into the patient's blood vessel by way of respective lumens provided in the connector 72, the tube 7, the outer needle hub 3, and the outer needle 2. In this instance as well, the hole 811 in the seal section body 81 is closed (shut off), and the sealing function performed thereby is maintained, as mentioned above. This ensures that leakage of liquid from the base end of the outer needle hub 3 does not occur, and further, sterility inside the outer needle hub 3 and the infusion line can be secured.

### <Second Embodiment>

A second embodiment of the indwelling needle assembly according to the present invention will be described below.

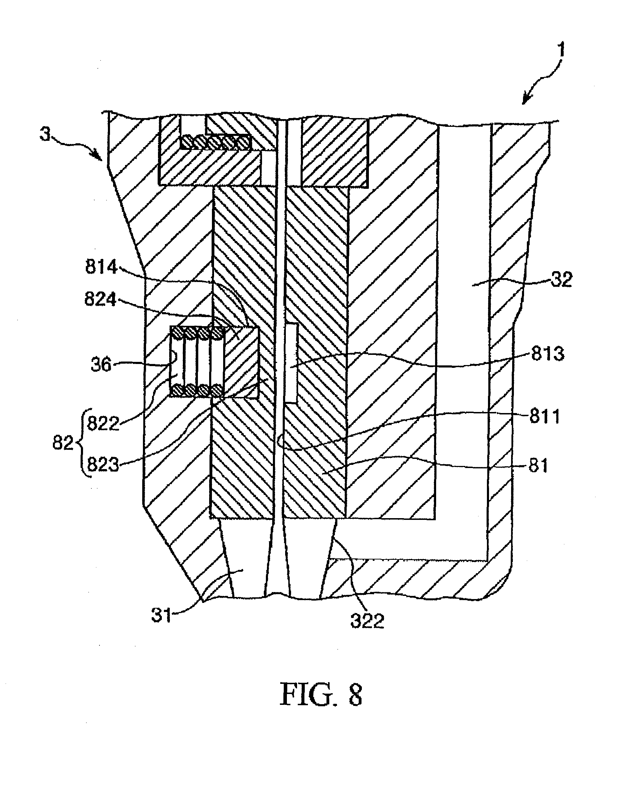

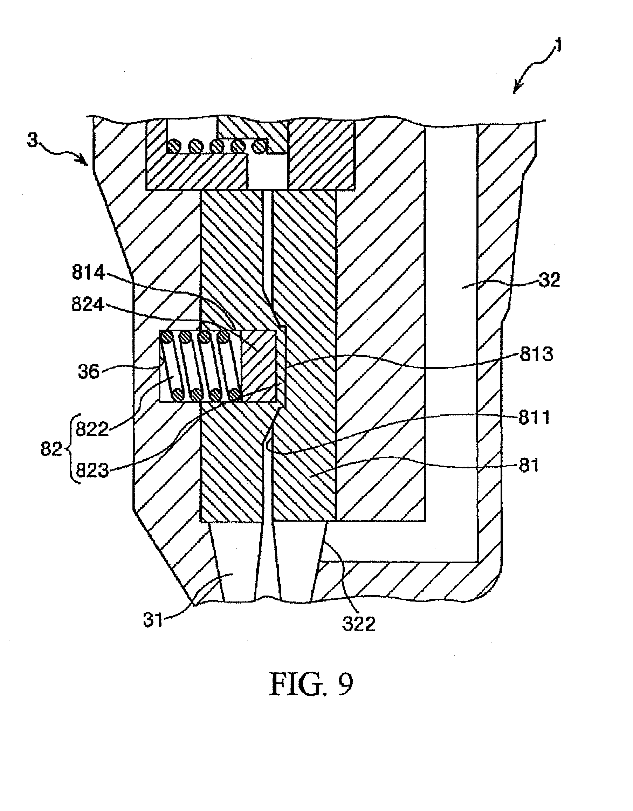

FIGS. 8 and 9 are longitudinal sectional views showing, in enlarged form, a tip part of an outer needle hub, which is possessed by the indwelling needle assembly according to the second embodiment. Incidentally, in the following descriptions, the upper side in FIGS. 8 and 9 will be referred to as "the base end," whereas the lower side will be referred to as "the tip."

An indwelling needle assembly according to the second embodiment will now be described, while focusing on differences from the indwelling needle assembly of the aforementioned first embodiment. Descriptions of the same items that have already been mentioned above will be omitted.

The indwelling needle assembly 1 according to the second embodiment is the same as the indwelling needle assembly 1 of the aforementioned first embodiment, except for differences in the configuration of the opening/closing means 82.

As shown in FIG. 8, a seal section body 81 is provided with a recess 814 in an outer peripheral surface thereof, and the seal section body 81 is thinned at that part. In this embodiment, the thinned part of the seal section body 81 constitutes a deformable section 823, which opens and closes a hole 811 by means of deformation.

In addition, in the recess 814, a flat plate-like pushing member 824 is provided between a coil spring 822 and the deformable section 823. As a result of this configuration, the deformable section 823 is pushed by the coil spring 822 through the pushing member 824.

The pushing member 824 preferably is formed from a hard material. This ensures that the deformable section 823 can more assuredly be deformed under a pushing action by the coil spring 822. Particularly, when the pushing member 824 is formed in a flat plate-like shape, the deformable section 823 can be pushed evenly, and the sealing function of the seal section 8 can be enhanced.

Examples of hard materials, other than the hard resin materials mentioned above, include various ceramic materials and various metallic materials.

In an assembled condition of the indwelling needle assembly 1 (i.e., in the condition shown in FIG. 8), the deformable section 823 is in a first state, such that an inner needle 4 can be inserted into the hole 811 in the seal section body 81, and an open condition is provided in which the hole 811 thereof is opened. Further, in such an open condition, the coil spring 822 is in a compressed state as a result of being pushed by the inner needle 4, whereas the deformable section 823 makes contact with the outside surface of the inner needle 4, and is restrained from deforming toward the side of the hole 811.

On the other hand, when the inner needle 4 is evulsed from the hole 811 in order to obtain a disassembled condition (the condition shown in FIG. 9), the restraint on deformation of the deformable section 823 is released, and the coil spring 822 expands. Consequently, the deformable section 823 is deformed toward the side of the hole 811 as a result of being pushed through the pushing member 824, and the right-hand surface thereof comes into abutment against (in firm contact with) the inside surface of the seal section body 81, thereby developing a closed condition in which the hole 811 is closed. This ensures that, in the disassembled condition, a liquid such as blood or liquid medicine can be securely prevented from flowing out from the base end of the outer needle hub 3 by way of the hole 811 in the seal section body 81. In addition, sterility at the inside (inside 31) of the outer needle hub 3 can be maintained.

Further, in such a closed condition, the deformable section 823 is partially contained within the recess 813, as shown in FIG. 9.

According to the indwelling needle assembly 1 of the second embodiment, operations and effects equivalent to those of the indwelling needle assembly 1 of the first embodiment above can be obtained.

Especially, in this embodiment, the deformable section 823 is formed on a portion of the seal section body 81, so that leakage of liquid from the base end of the outer needle hub 3, by way of a gap, if any, occurring between the deformable section 823 and the seal section body 81, can be excluded.

In addition, it is preferable to form the deformable section 823 and the seal section body 81 as one body, from the viewpoint of reducing the number of component parts making up the indwelling needle assembly 1.

Incidentally, although the pushing member 824 may be provided as required, the pushing member 824 also may be omitted if desired.

While the indwelling needle assembly according to the present invention has been described above referring to the embodiments shown in the drawings, the invention is not limited to such embodiments. Sections and parts constituting the indwelling needle assembly can be replaced by other parts of arbitrary configurations, whereby functions equivalent to the above-mentioned functions can be obtained. In addition, other arbitrary components can be added to the indwelling needle assembly.

Further, in the present invention, two or more arbitrary configurations from the first and second embodiments can be adopted in combination with each other.

In addition, the indwelling needle assembly according to the present invention is not limited to being used in a state of insertion into a blood vessel. The indwelling needle assembly may also be used in a state of being inserted into an abdominal cavity, a thoracic cavity, a lymph vessel, a vertebral column, or the like.

In addition, a cap may be provided, which is attached to a base end part of the outer needle hub after the inner needle has been evulsed from the outer needle. This ensures that leakage of liquid from the base end of the outer needle hub can be prevented more assuredly.

In this case, the cap may be formed as a unitary body together with the outer needle hub, or may be a body which is separated from the outer needle hub. In addition, the cap may be fixed to the outer needle hub by any method, for example, a friction fixing method, a method of fixing by hooking, or the like.

Further, the protector is not limited to the protector that is shown in the drawings. The protector may be turnable (displaceable) between a position that covers at least the point of the inner needle, and a position separated from the inner needle.

In addition, the connector provided at the end part of the tube is not particularly limited. Examples of connectors which can be used with the present invention include a three-way cock and a needleless connector, as described in Japanese Laid-Open Patent Publication No. 2005-261931.

Moreover, the member provided at the end part of the tube is not limited to the above-mentioned connector, and may be, for example, a cap, an air filter, or the like.

Further, in the indwelling needle assembly according to the present invention, the connector, the cap, and the air filter, as mentioned above, may be exchangeable with each other when attached to the end part of the tube.

### INDUSTRIAL APPLICABILITY

The indwelling needle assembly according to the present invention includes an inner needle having a sharp point at a tip portion thereof, an inner needle hub fixed to a base end part of the inner needle, a hollow outer needle in which the inner needle is inserted, an outer needle hub fixed to a base end part of the outer needle and having a joint section to which a tube is connected, and a seal section provided in the outer needle hub. The seal section includes a seal section body having a hole into which the inner needle is insertable, and opening/closing means having a shutter member for opening and closing the hole, together with an urging member for urging the shutter member in a direction to close the hole. Therefore, the hole in the seal section body, through which the inner needle has passed in an assembled condition with the inner needle inserted into the outer needle, is forcibly closed by the opening/closing means. Therefore, leakage of liquid, such as blood or a liquid medicine, from the base end of the outer needle hub can be securely prevented from occurring in the disassembled condition, where the inner needle has been evulsed from the outer needle. Accordingly, the indwelling needle assembly of the present invention has industrial applicability.

## Claims

1. An indwelling needle assembly comprising:
an inner needle having a sharp needlepoint at a tip portion thereof;
an inner needle hub fixed to a base end part of the inner needle;
a hollow outer needle in which the inner needle is inserted;
an outer needle hub fixed to a base end part of the outer needle and having a joint section to which a tube is connected; and
a seal section provided in the outer needle hub,
wherein the seal section includes
a seal section body having a hole into which the inner needle can be inserted, and
opening/closing means having a shutter member for opening and closing the hole, and an urging member for urging the shutter member in a direction to close the hole.

2. The indwelling needle assembly as set forth in claim 1, wherein the shutter member is provided in a space formed at an intermediate part of the hole.

3. The indwelling needle assembly as set forth in claim 1 or 2,
wherein the seal section body has a recess, which is provided to confront the hole and to face the shutter member, and
the shutter member is partially contained within the recess when the hole is in a closed state.

4. An indwelling needle assembly comprising:
an inner needle having a sharp needlepoint at a tip portion thereof;
an inner needle hub fixed to a base end part of the inner needle;
a hollow outer needle in which the inner needle is inserted;
an outer needle hub fixed to a base end part of the outer needle and having a joint section to which a tube is connected; and
a seal section provided in the outer needle hub,
wherein the seal section includes
a seal section body having a hole into which the inner needle can be inserted, and
opening/closing means provided in the seal section body and having a deformable section for opening and closing the hole through deformation thereof, and an urging member for urging the deformable section in a direction to close the hole.

5. The indwelling needle assembly as set forth in claim 4, further comprising:
a pushing member for pushing the deformable section, between the deformable section and the urging member,
wherein the deformable section is pushed by the urging member through the pushing member.

6. The indwelling needle assembly as set forth in claim 4 or 5,
wherein the seal section body has a recess which is provided to confront the hole and to face the deformable section, and
the deformable section is partially contained within the recess when the hole is in a closed state.

7. The indwelling needle assembly as set forth in claim 1 or 4, wherein the outer needle hub comprises a containing section containing at least a portion of the urging member therein.

8. The indwelling needle assembly as set forth in claim 1 or 4, wherein the urging member includes an elastically deformable spring.

9. The indwelling needle assembly as set forth in claim 8, wherein the spring is a coil spring.

10. The indwelling needle assembly as set forth in claim 1 or 4, wherein the seal section body is made of an elastic material.
